# FASCICULE DE BREVET EUROPEEN

(11) **EP 2 694 027 B1**
(45) Date de publication et mention de la délivrance du brevet: **08.07.2015**
(21) Numéro de dépôt: 12718283.0
(22) Date de dépôt: 30.03.2012
(51) Int. Cl.: A61K 8/37, A61Q 19/00

(54) **UTILISATION DE COMPOSÉS (ÉTHOXY-HYDROXYPHÉNYL)ALKYLCÉTONE OU ÉTHOXYHYDROXYALKYLPHÉNOL POUR TRAITER LA PEAU GRASSE**
VERWENDUNG VON (ETHOXY-HYDROXY-PHENYL)ALKYLKETON- ODER ETHOXY-HYDROXY -ALKYLPHENOLVERBINDUNGEN ZUR BEHANDLUNG VON FETTIGER HAUT
USE OF (ETHOXY-HYDROXY PHENYL)ALKYL KETONE OR ETHOXY HYDROXY ALKYL PHENOL COMPOUNDS FOR TREATING OILY SKIN

(30) Priorité: 01.04.2011 FR 1152794
(43) Date de publication de la demande: 12.02.2014
(73) Titulaire: L'Oréal, 75008 Paris (FR)
(72) Inventeur: DALKO, Maria, F-78000 Versailles (FR)
(74) Mandataire: Brohmi, Karim
(86) Numéro de dépôt international: PCT/FR2012/050689
(87) Numéro de publication internationale: WO 2012/131272

(56) Documents cités:
- FR-A1- 2 943 896

## Description

La présente invention concerne un procédé cosmétique pour traiter et/ou prévenir les peaux grasses où à tendance grasse et/ou les défauts esthétiques cutanés associés, comprenant l'application topique sur la peau, d'une composition comprenant dans un milieu cosmétiquement acceptable au moins un composé 4-(3-éthoxy-4-hydroxyphényl)alkylcétone ou 2-éthoxy 4-hydroxyalkylphénol de formule (I) susceptibles d'être dérivés de vanilline.

L'invention concerne également l'utilisation cosmétique desdits composés comme agents pour traiter et/ou prévenir les peaux grasses où à tendance grasse et/ou les défauts esthétiques cutanés associés.

Par « la peau», on entend l'ensemble de la peau du corps, incluant le cuir chevelu et les muqueuses.

Le sébum constitue normalement un agent hydratant de l'épiderme et peut être impliqué dans l'homéostasie de l'épiderme, et notamment dans la prolifération et/ou la différenciation des cellules épidermiques.

Il est le produit naturel de la glande sébacée qui constitue une annexe de l'unité pilosébacée. Il s'agit essentiellement d'un mélange plus ou moins complexe de lipides. Classiquement, la glande sébacée produit du squalène, des triglycérides, des cires aliphatiques, des cires de cholestérol et, éventuellement du cholestérol libre (Stewart, M. E., Semin Dermatol 11, 100-105(1992)). L'action des lipases bactériennes convertit une part variable des triglycérides formés en acides gras libres.

Le sébocyte constitue la cellule compétente de la glande sébacée. La production de sébum est associée à un programme de différenciation terminale de cette cellule. Durant cette différenciation, l'activité métabolique du sébocyte est essentiellement axée sur la biosynthèse des lipides (la lipogénèse) et plus précisément sur la néosynthèse d'acide gras.

Une peau grasse ou hyper-séborrhéique est caractérisée, notamment, par une sécrétion et une excrétion exagérées de sébum. Classiquement, un taux de sébum supérieur à 200 µg/cm² mesuré au niveau du front est considéré comme caractéristique d'une telle peau grasse.

Une telle peau est en outre souvent associée à un défaut de desquamation, un teint luisant, un grain de peau épais, des pores dilatés, ou un relief irrégulier, manifestations ressenties comme des imperfections cutanées ou défauts esthétiques. L'apparence et/ou la visibilité des pores est aussi une caractéristique de la peau grasse. La brillance de la peau est aussi liée à la dilatation des pores, d'où l'intérêt de trouver des actifs de réduire la taille des pores dilatés.

Pour lutter contre l'hyperséborrhée, il a déjà été proposé divers composés qui, par application topique sur la peau, sont susceptibles de diminuer la lipogenèse au niveau des sébocytes et limiter, par voie de conséquence, la production de sébum. Les traitements actuellement disponibles à l'égard de l'hyperséborrhée ne sont pas totalement satisfaisants, notamment au regard des effets secondaires qui leur sont fréquemment associés, tels qu'irritatifs avec certains topiques comme les rétinoïdes et benzoylperoxydes.

FR1 943 896 divulgue un "roll-on" pour l'application d'une composition cosmétique pour le soin des peaux grasses et/ou acnéiques et/ou séborrhéiques. Le document divulgue que les compositions peuvent contenir en plus des actifs anti-séborrhéiques, entre eux un mélange d'extraits de pimprenelle, gingembre et cannelier qui est vendu sous la dénomination Sebustop^{R}.

Il demeure donc un besoin de disposer de nouveaux actifs susceptibles d'exercer une action cosmétique bénéfique sur les peaux grasses ou à tendance grasse et/ou les défauts esthétiques associés.

Il demeure également un besoin de disposer de nouveaux actifs susceptibles d'exercer une action cosmétique bénéfique sur les états du cuir chevelu gras.

La présente invention a pour objet de satisfaire ces besoins.

La demanderesse a maintenant découvert de manière surprenante et inattendue que l'utilisation d'au moins un composé de formule (I) selon l'invention pouvait s'avérer utile pour prévenir et/ou traiter, de manière efficace et sans les inconvénients ci-dessus cités, des peaux grasses ou à tendance grasse et/ou des défauts esthétiques cutanés associés.

Cette découverte constitue la base de l'invention.

La présente invention a donc pour objet un procédé cosmétique pour traiter et/ou prévenir les peaux grasses où à tendance grasse et/ou les défauts esthétiques cutanés associés, comprenant l'application topique sur la peau, d'une composition comprenant dans un milieu cosmétiquement acceptable au moins un composé de formule (I) : 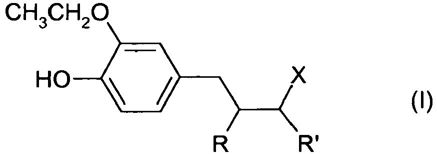 dans laquelle :
- R représente un atome d'hydrogène, ou un radical hydrocarboné, saturé ou insaturé, linéaire ou ramifié, en C1-C6;
- R' représente un radical hydrocarboné, saturé ou insaturé, linéaire ou ramifié, en C1-C18, éventuellement substitué par un groupe hydroxyle;
- C-X représente C=O ou CH-OH.

La présente invention a également pour objet l'utilisation cosmétique d'au moins un composé de formule (I) 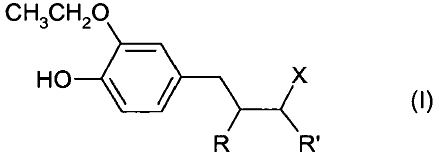 dans laquelle :
- R représente un atome d'hydrogène, ou un radical hydrocarboné, saturé ou insaturé, linéaire ou ramifié, en C1-C6;
- R' représente un radical hydrocarboné, saturé ou insaturé, linéaire ou ramifié, en C1-C18, éventuellement substitué par un groupe hydroxyle;
- C-X représente C=O ou CH-OH ;
   comme agent pour traiter et/ou prévenir les peaux grasses où à tendance grasse et/ou les défauts esthétiques cutanés associés.

Selon cette utilisation le composé de formule (I) est avantageusement contenu dans une composition comprenant un milieu cosmétiquement acceptable.

Au sens de la présente invention, on entend par « prévenir » le fait de réduire le risque ou la probabilité de manifestation d'un phénomène donné.

### Indications

Un composé de l'invention peut avantageusement être utilisé en vue de traiter et/ou prévenir la peau grasse ou à tendance grasse et/ou les signes cutanés associés.

L'invention vise l'ensemble de la peau du corps, incluant le cuir chevelu, et de manière préférée, la peau du visage, du décolleté, du cou, des bras et avant-bras, voire de manière plus préférée encore, la peau du visage (en particulier du front, nez, joues, menton), du décolleté et du cou.

Selon un mode de réalisation, un défaut esthétique cutané peut être choisi parmi des imperfections cutanées dues à une hyperséborrhée et/ou un désordre du cuir chevelu.

Les signes cutanés esthétiques d'une hyperséborrhée, ou peau grasse, plus particulièrement considérés par l'invention peuvent être une peau luisante, et/ou épaisse et/ou dont les orifices folliculaires ou les pores sont dilatés, voire dans certains cas sont comblés de minuscules spicules cornés ou par des comédons. Une peau grasse est souvent associée à un défaut de desquamation, et à un grain de peau épais.

Les signes cutanés esthétiques, ou imperfections, d'une peau grasse ou à tendance grasse peuvent en particulier être choisis parmi un grain de peau épais, une peau luisante ou brillante, une peau présentant des orifices folliculaires ou des pores dilatés, une peau présentant des orifices folliculaires ou des pores comblés de spicules cornés ou de comédons, une peau rugueuse ou présentant un relief irrégulier, ou une peau présentant une altération du teint.

Une peau grasse ou à tendance grasse peut également présenter des altérations du teint de la peau tel qu'un teint brouillé, inhomogène, ou terne.

### Procédé cosmétique

L'invention concerne un procédé cosmétique dédié en particulier aux individus présentant une peau grasse ou à tendance grasse et/ou des défauts esthétiques cutanés associés.

Un individu concerné par un procédé de traitement cosmétique de l'invention est naturellement un individu présentant, ou susceptible de présenter, au moins une des indications de soins cosmétiques précédemment définies.

Un procédé de l'invention permet de traiter la peau grasse ou à tendance grasse, et en particulier un défaut esthétique de la peau tel que défini précédemment.

De manière préférée, un procédé selon l'invention comprendra l'application topique d'une composition selon l'invention sur la peau du visage, et/ou le cuir chevelu.

Le procédé selon l'invention peut s'avérer tout particulièrement utile :
- pour prévenir et/ou traiter les défauts esthétiques de la peau grasse ou à tendance grasse,- pour prévenir et/ou traiter une peau présentant des orifices folliculaires ou des pores dilatés, en particulier pour réduire l'apparence et/ou la visibilité des pores, notamment pour resserrer les pores et/ou diminuer la taille des pores, et/ou diminuer le nombre de pores visibles,
- pour prévenir et/ou traiter une peau présentant des orifices folliculaires ou des pores comblés de spicules cornés ou de comédons,
- pour prévenir et/ou traiter une peau rugueuse ou présentant un relief irrégulier,
- pour prévenir et/ou traiter une altération du teint de la peau, tel qu'un teint brouillé, inhomogène, ou terne,
- pour prévenir et/ou traiter une peau luisante ou brillante (réduire la brillance de la peau),
- pour prévenir et/ou traiter un défaut esthétique du cuir chevelu lié à un excès d'excrétion et/ou de sécrétion de sébum, ou
- pour améliorer le confort des peaux ou des cuirs chevelus.

Toutes ces indications peuvent être mesurées aisément pat l'homme du métier, selon des techniques bien connues.

L'apparence et/ou la visibilité des pores peut être mesurée par des techniques connues de l'homme du métier, et notamment selon les indications publiées dans la demande WO2011/114010. A titre d'exemple, la taille des pores peut être mesurée, à l'aide du dispositif Dermascore^{®} selon le protocole décrit dans l'article : Quantification of facial pores using image analysis Ghislain François et al, Cosmetic dermatology, vol 22,n ° 9, pages 457-463.

La mesure de l'apparence et/ou la visibilité des pores peut encore être effectuée par mesure de la taille des pores sur le front à différents jours du traitement sur chaque hémivisage traité avec une composition comprenant l'actif (versus hémivisage traité avec placebo), en notant à l'aide d'une échelle à 4 valeurs :
Valeur = 0 : pas de pores observés
Valeur = 1 : pores de taille petite observés
Valeur = 2 : pores de taille moyenne observés
Valeur = 3 : pores de taille grande observés

On peut ainsi calculer la différence de valeurs Δ obtenue entre la valeur notée pour l'hémivisage traité avec l'actif et celle notée pour l'hémivisage traité avec le placébo.

La luisance ou brillance de la peau peut être mesurée par des techniques connues de l'homme du métier, et notamment selon les indications publiées dans les demandes FR2881643B1 et FR2952299B1. A titre d'exemple, la luisance peut être évaluée visuellement, sur le front, à différents jours du traitement (total de 45 jours) sur chaque hémivisage traité avec une composition comprenant l'actif (versus hémivisage traité avec placebo), en notant à l'aide d'une échelle à 4 valeurs :
Valeur = 0 : pas de luisance observée
Valeur = 1 : luisance faible
Valeur = 2 : luisance moyenne
Valeur = 3 : luisance forte.

On peut ainsi calculer la différence de valeurs Δ obtenue entre la valeur notée pour l'hémivisage traité avec l'actif et celle notée pour l'hémivisage traité avec le placébo. L'effet matifiant (moins brillant) peut aussi être évalué à l'aide d'un gonioréflectomètre, en mesurant le rapport R entre la réflexion spéculaire et la réflexion diffuse. Une valeur de R inférieure ou égale à 2 traduit généralement un effet matifiant.

Le teint de la peau peut être mesuré par des techniques connues de l'homme du métier, et notamment selon les indications publiées dans l'exemple 1 de la demande WO2011/070508. A titre d'exemple, la luminosité du teint du visage peut être évaluée visuellement par un dermatologue investigateur après application de la composition comprenant l'actif versus placebo, selon une échelle de 0 à 4 (0= très éclatant ; 1= éclatant ; 2= ni terne/ni éclatant ; 3= terne ; 4= très terne).

### Composés de formule (I)

Les composés selon l'invention répondent donc à la formule (I) : 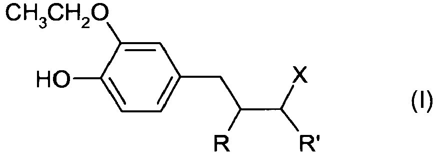 dans laquelle :
- R représente un atome d'hydrogène, ou un radical hydrocarboné, saturé ou insaturé (alkyle ou alcényle), linéaire ou ramifié, en C1-C6;
- R' représente un radical hydrocarboné, saturé ou insaturé (alkyle ou alcényle), linéaire ou ramifié, en C1-C18, éventuellement substitué par un groupe hydroxyle; - C-X représente C=O ou CH-OH.

De préférence, R représente H, méthyle ou éthyle.

De préférence, R' représente un radical hydrocarboné linéaire, saturé en C1-C6 ou insaturé en C2-C6, éventuellement substitué par un groupe hydroxyle.

De préférence, les composés répondent à la formule (I), dans laquelle :
- C-X représente C=O, R=H et R' représente un radical alkyle linéaire en C1-C6, éventuellement substitué par un OH; de préférence R'= méthyle ou éthyle; ou bien
- C-X représente CH-OH, R=H et R' représente un radical alkyle linéaire en C1-C6, éventuellement substitué par un OH; de préférence R'= méthyle ou éthyle.

On peut particulièrement citer les composés (1), (2) et (3) suivants : 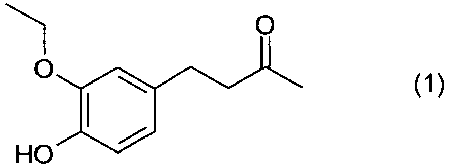 4-(3-éthoxy-4-hydroxyphényl)butan-2-one 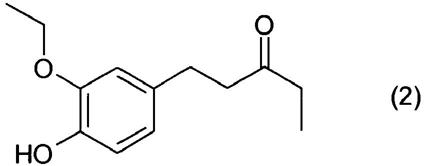 4-(3-éthoxy-4-hydroxyphényl)pentan-3-one 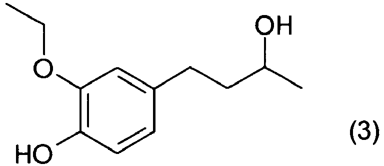 2-éthoxy-4-(3-hydroxybutyl)phénol

On préférera le composé (1) : 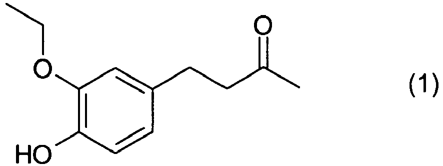 4-(3-éthoxy-4-hydroxyphényl)butan-2-one

On peut bien évidemment utiliser un mélange de composés de formule (I).

Les composés de formule (I) peuvent être préparés aisément par l'homme du métier sur la base de ses connaissances générales. On peut notamment citer les références bibliographiques suivantes : J. Asian Natural Products Research, 2006, 8(8), 683-688; Helv. Chimica Acta, 2006, 89(3), 483-495; Chem. Pharm. Bull., 2006, 54(3), 377-379; et Bioorg. Med. Chem. Lett., 2004, 14(5), 1287-1289.

Ils peuvent ainsi être préparés à partir d'éthylvanilline de la manière suivante :

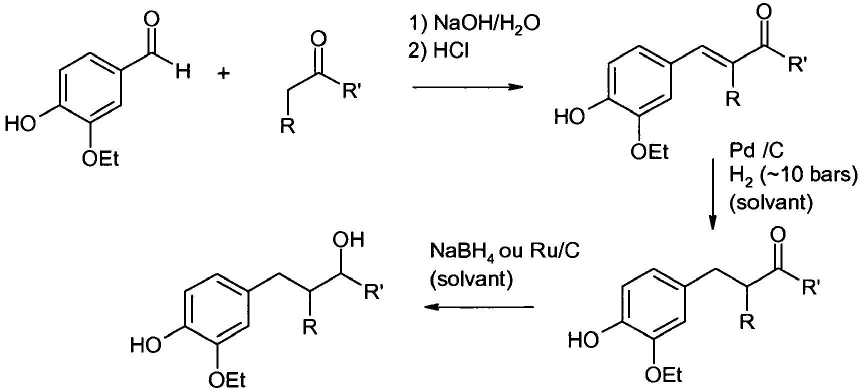

Les composés de formule (I) avec C-X représentant CHOH peuvent être obtenus par réduction des composés correspondants dans lesquels C-X représente C=O, par exemple par réduction avec Ru/C ou NaBH₄.

Les composés de formule (I), seul ou en mélange, peuvent être employés à raison de 0,1 à 10% en poids, notamment 0,5 à 5% en poids, par rapport au poids de la total de la composition les contenant.

### Composition

La composition selon la présente invention est avantageusement cosmétique.

La composition pourra être une composition de soin et/ou de nettoyage et/ou de gommage et/ou de maquillage, destinée à être rincée ou non rincée. De préférence, il s'agira d'une composition de soin non rincée. Lorsque la composition comprendra des agents exfoliants ou charges abrasives, elle sera de préférence rincée.

Les compositions selon l'invention sont avantageusement destinées à une application topique sur le visage (en particulier le front, le nez, les joues, le menton), et/ou le cuir chevelu.

La composition selon l'invention contient un milieu cosmétiquement acceptable, c'est-à-dire un milieu compatible avec la peau, les ongles, les muqueuses, les tissus et les cheveux, sans odeur, couleur ou aspect désagréable, et qui ne génère pas de picotement, tiraillement ou rougeur inacceptable pour l'utilisateur.

De préférence, ledit milieu comprend de l'eau et/ou un ou plusieurs solvants organiques cosmétiquement acceptables. Les solvants organiques peuvent être choisis parmi les monoalcools, linéaires ou ramifiés, en C1-C6 tels que l'éthanol, l'isopropanol, le tertio-butanol ou le n-butanol; les polyols tels que le glycérol, le propylèneglycol, l'hexylène glycol (ou 2-méthyl-2,4-pentanediol), et les polyéthylèneglycols; les éthers de polyols comme le monométhyléther de dipropylèneglycol; et leurs mélanges.

De préférence, la composition cosmétique utilisée selon l'invention comprend une quantité de solvants organiques allant de 0,05% à 60%, de préférence de 0,5 à 50%, et mieux encore de 1 à 40% en poids, par rapport au poids total de la composition cosmétique.

Selon un mode préféré de réalisation de l'invention, la composition a un pH de préférence proche de celui de la peau, compris entre 4 et 7.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des vésicules lipidiques de type ionique et/ou non ionique (liposomes, niosomes, oléosomes). Ces compositions sont préparées selon les méthodes usuelles.

En outre, la composition selon l'invention peut être plus ou moins fluide et avoir l'aspect d'une crème blanche ou colorée, d'une pommade, d'un lait, d'une lotion, d'un sérum, d'une pâte, d'un gel moussant, d'un soin, d'un tonique ou d'une mousse. Elle peut être éventuellement appliquée sur la peau sous forme d'aérosol. Elle peut aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R'COOR² et R'OR² dans laquelle R' représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldo-décyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les poly-diméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par huile hydrocarbonée dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations KSG par la société Shin-Etsu, sous les dénominations "Trefil", "BY29" ou "EPSX" par la société Dow Corning ou sous les dénominations "Gransil" par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination "DC 5225 C" par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004, et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

La composition selon l'invention peut contenir également des adjuvants habituels dans le domaine cosmétique, tels que les gélifiants hydrophiles ou lipophiles, les conservateurs, l'eau, les solvants, les parfums, les charges, les filtres UV, les absorbeurs d'odeur, les matières colorantes, les agents basiques, les acides, les tensioactifs non ioniques, anioniques, cationiques.

Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges.

Comme gélifiants hydrophiles ou lipophiles, on peut citer notamment les carbopol, les luvigel, l'Hostacerin AMPS, le Simulgel, les Sepigel, les gommes de xanthane, de guar, de cellulose, les alginates et leurs mélanges. On peut citer aussi les hectorites.

Bien entendu, l'homme du métier veillera à choisir ce ou ces éventuels ingrédients et/ou actifs complémentaires, et/ou leur quantité, de manière telle que les propriétés avantageuses du composé de formule (I) selon l'invention, ne soient pas, ou substantiellement pas, altérées par l'adjonction envisagée.

Les compositions selon l'invention pourront être appliquées directement sur la peau ou, de façon alternative, sur des supports cosmétiques de type occlusif ou non occlusif, destinés à être appliqués de façon localisée sur la peau. A titre d'exemples de supports cosmétiques, non limitatifs, on peut notamment citer un patch, une lingette, un roll-on et un stylo.

La composition selon la présente invention comprendra selon un mode de réalisation particulier, outre le composé de formule (I), au moins un actif additionnel pour le soin des peaux grasses ou à tendance grasse.

### Actifs additionnels pour le soin des peaux grasses :

Par l'expression « actif additionnel de soin des peaux grasses », on entend, dans le cadre de la présente invention, un composé qui a par lui-même, c'est-à-dire ne nécessitant pas l'intervention d'un agent extérieur pour l'activer, une activité biologique qui peut être en particulier :
- une activité desquamante (qui permet l'ouverture des comédons), et/ou
- une activité anti-microbienne (notamment sur *P. acnes*), et/ou
- une activité apaisante ou anti-inflammatoire, et/ou
- une activité sébo-régulatrice, et/ou
- une activité anti-oxydante (qui empêche l'oxydation du squalène et la formation des comédons)
- une activité cicatrisante ;
- une activité astringente.

L'actif additionnel pour le soin des peaux grasses utilisable dans les compositions de l'invention est préférentiellement choisi parmi les agents desquamants, les agents antimicrobiens, les agents apaisants, les agents anti-inflammatoires, les agents sébo-régulateurs, les agents antioxydants, les agents cicatrisants, les agents astringents, et leurs mélanges.

L'actif additionnel pour le soin des peaux grasses utilisé dans la composition selon l'invention peut représenter de 0,0001 % à 20 %, de préférence de 0,01 % à 10 % et mieux encore, de 0,01 % à 5 % en poids par rapport au poids total de la composition.

Selon un mode de réalisation, une composition selon l'invention ne comprendra pas d'huile essentielle.

L'invention est illustrée plus en détail dans les exemples suivants.

Les exemples qui suivent illustrent l'invention sans en limiter la portée. Les composés sont, selon le cas, cités en noms chimiques ou en noms CTFA (International Cosmetic Ingredient Dictionary and Handbook).

### Exemple : Lotion traitante pour peaux grasses :

Les quantités sont données en pourcentage massique par rapport au poids total de la composition.

| | |
|---|---|
| Vitamine E | 0.05 % |
| Myrtrimonium bromide | 0.03 % |
| Disodium EDTA | 0.08 % |
| PEG-8 | 5.0 % |
| Glycerine | 3.0 % |
| PEG-60 hydrogenated castor oil | 0.5 % |
| 4-(3-éthoxy-4-hydroxyphényl)butan-2-one (Composé (1) selon l'invention) | 2 % |
| N-lauroylsarcosinate d'isopropyl* | 1.4 % |
| Conservateurs | 0.8 % |
| Parfum | 0.08 % |
| Eau | QSP 100 % |

| | |
|---|---|
| * Eldew® SL 205 de chez Ajinomoto | |

Cette composition, appliquée sur la peau, permet de traiter et/ou prévenir les peaux grasses où à tendance grasse et/ou les défauts esthétiques cutanés associés tels que décrits dans la présente demande.

## Revendications

1. Procédé cosmétique pour traiter et/ou prévenir les peaux grasses où à tendance grasse et/ou les défauts esthétiques cutanés associés, comprenant l'application topique sur la peau, d'une composition comprenant dans un milieu cosmétiquement acceptable au moins un composé de formule (I) : 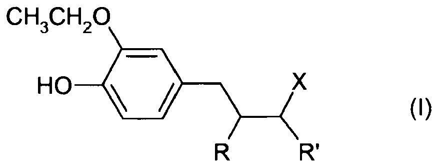 dans laquelle :
- R représente un atome d'hydrogène, ou un radical hydrocarboné, saturé ou insaturé, linéaire ou ramifié, en C1-C6;
- R' représente un radical hydrocarboné, saturé ou insaturé, linéaire ou ramifié, en C1-C18, éventuellement substitué par un groupe hydroxyle;
- C-X représente C=O ou CH-OH.

2. Procédé selon la revendication 1, dans laquelle R représente H, méthyle ou éthyle.

3. Procédé selon l'une des revendications précédentes, dans laquelle R' représente un radical hydrocarboné linéaire, saturé en C1-C6 ou insaturé en C2-C6, éventuellement substitué par un groupe hydroxyle.

4. Procédé selon l'une des revendications précédentes, dans laquelle les composés répondent à la formule (I), dans laquelle :
- C-X représente C=O, R=H et R' représente un radical alkyle linéaire en C1-C6, éventuellement substitué par un OH; de préférence R'= méthyle ou éthyle; ou bien
- C-X représente CH-OH, R=H et R' représente un radical alkyle linéaire en C1-C6, éventuellement substitué par un OH; de préférence R'= méthyle ou éthyle.

5. Procédé selon l'une des revendications précédentes, dans laquelle les composés de formule (I) sont choisis parmi les composés suivants : 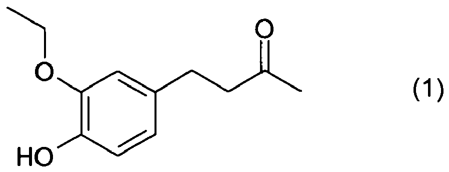 4-(3-éthoxy-4-hydroxyphényl)butan-2-one 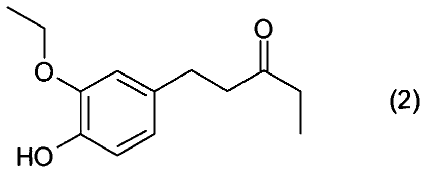 4-(3-éthoxy-4-hydroxyphényl)pentan-3-one 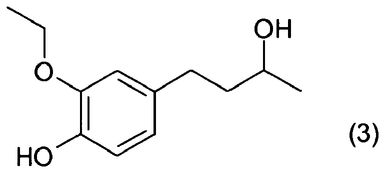 2-éthoxy-4-(3-hydroxybutyl)phénol

6. Procédé selon la revendication 5, dans laquelle le composé de formule (I) est

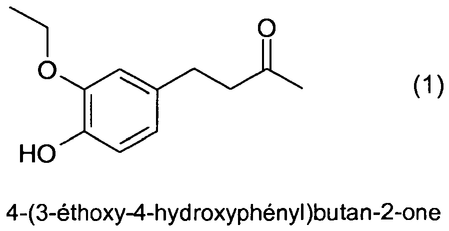

7. Procédé selon l'une des revendications précédentes, dans laquelle le composé de formule (I), seul ou en mélange, est présent à raison de 0,1 à 10% en poids, notamment 0,5 à 5% en poids, par rapport au poids total de la composition.

8. Procédé selon l'une quelconque des précédentes revendications, dans lequel les défauts esthétiques cutanés sont choisis parmi des imperfections cutanées dues à une hyperséborrhée, et/ou un désordre du cuir chevelu.

9. Procédé selon l'une quelconque des revendications précédentes, dans lequel les défauts esthétiques cutanés sont choisis parmi un grain de peau épais, une peau luisante ou brillante, une peau présentant des orifices folliculaires ou des pores dilatés, une peau présentant des orifices folliculaires ou des pores comblés de spicules cornés ou de comédons, une peau rugueuse ou présentant un relief irrégulier, ou une peau présentant une altération du teint.

10. Utilisation cosmétique d'au moins un composé de formule (I) tel que défini dans l'une des revendications 1 à 6, comme agent pour traiter et/ou prévenir les peaux grasses où à tendance grasse et/ou les défauts esthétiques cutanés associés.

11. Utilisation selon la revendication précédente, dans laquelle les défauts esthétiques cutanés sont tels que définis dans l'une des revendications 8 ou 9.

12. Utilisation selon l'une quelconque des revendications 10 et 11, où le composé de formule (I) est contenu dans une composition comprenant un milieu cosmétiquement acceptable.

## Patentansprüche

1. Kosmetisches Verfahren zur Behandlung und/oder Vorbeugung von fettiger Haut oder zu Fettigkeit neigender Haut und/oder den damit in Zusammenhang stehenden ästhetischen Hautmakeln, umfassend das topische Auftragen auf die Haut einer Zusammensetzung, die in einem kosmetisch akzeptablen Medium mindestens eine Verbindung der Formel (I) umfasst: 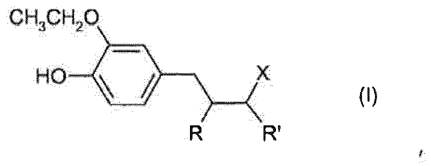 worin:
- R ein Wasserstoffatom oder einen gesättigten oder ungesättigten, geraden oder verzweigten C1-C6-Kohlenwasserstoffrest darstellt;
- R' einen gesättigten oder ungesättigten, geraden oder verzweigten C1-C18-Kohlenwasserstoffrest darstellt, der gegebenenfalls mit einer Hydroxylgruppe substituiert ist;
- C-X für C=O oder CH-OH steht.

2. Verfahren nach Anspruch 1, wobei R für H, Methyl oder Ethyl steht.

3. Verfahren nach einem der vorhergehenden Ansprüche, wobei R' einen geraden gesättigten C1-C6- oder ungesättigten C2-C6-Kohlenwasserstoffrest darstellt, der gegebenenfalls mit einer Hydroxylgruppe substituiert ist.

4. Verfahren nach einem der vorhergehenden Ansprüche, wobei die Verbindungen die Formel (I) besitzen, worin:
- C-X für C=O steht, R = H ist und R' einen geraden C1-C6-Alkylrest darstellt, der gegebenenfalls mit einem OH substituiert ist; wobei vorzugsweise R' = Methyl oder Ethyl; oder
- C-X für CH-OH steht, R = H ist und R' einen geraden C1-C6-Alkylrest darstellt, der gegebenenfalls mit einem OH substituiert ist; wobei vorzugsweise R' = Methyl oder Ethyl.

5. Verfahren nach einem der vorhergehenden Ansprüche, wobei die Verbindungen der Formel (I) aus den folgenden Verbindungen ausgewählt werden: 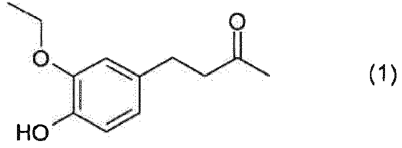 4-(3-Ethoxy-4-hydroxyphenyl)butan-2-on 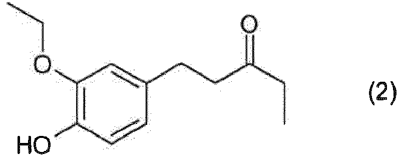 1-(3-Ethoxy-4-hydroxyphenyl)pentan-3-on 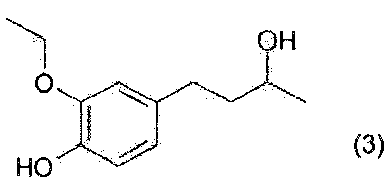 2-Ethoxy-4-(3-hydroxybutyl)phenol

6. Verfahren nach Anspruch 5, wobei es sich bei der Verbindung der Formel (I) um Folgende handelt: 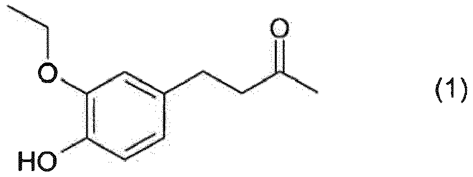 4-(3-Ethoxy-4-hydroxyphenyl)butan-2-on

7. Verfahren nach einem der vorhergehenden Ansprüche, wobei die Verbindung der Formel (I), allein oder im Gemisch, zu 0,1 bis 10 Gew.-%, insbesondere zu 0,5 bis 5 Gew.-%, bezogen auf das Gesamtgewicht der Zusammensetzung, vorliegt.

8. Verfahren nach einem der vorhergehenden Ansprüche, wobei die ästhetischen Hautmakel aus Hautmängeln aufgrund einer Hyperseborrhöe und/oder einer Störung der Kopfhaut ausgewählt werden.

9. Verfahren nach einem der vorhergehenden Ansprüche, wobei die ästhetischen Hautmakel aus einer dicken Hautstruktur, scheinender oder glänzender Haut, Haut mit erweiterten Follikelkanälen oder Poren, Haut, deren Follikelkanäle oder Poren mit verhornten Nadeln oder Komedonen verstopft sind, rauer Haut oder Haut mit unregelmäßiger Oberflächenstruktur oder Haut mit einer Veränderung des Hauttons ausgewählt werden.

10. Kosmetische Verwendung mindestens einer Verbindung der Formel (I) nach einem der Ansprüche 1 bis 6 als Mittel zur Behandlung und/oder Vorbeugung von fettiger Haut oder zu Fettigkeit neigender Haut und/oder den damit in Zusammenhang stehenden ästhetischen Hautmakeln.

11. Verwendung nach dem vorhergehenden Anspruch, wobei die ästhetischen Hautmakel wie in einem der Ansprüche 8 oder 9 definiert sind.

12. Verwendung nach einem der Ansprüche 10 und 11, wobei die Verbindung der Formel (I) in einer Zusammensetzung enthalten ist, die ein kosmetisch akzeptables Medium umfasst.

## Claims

1. Cosmetic method for treating and/or preventing greasy skin or greasiness-prone skin and/or the associated cutaneous aesthetic defects, comprising the topical application to the skin of a composition comprising, in a cosmetically acceptable medium, at least one compound of formula (I) : 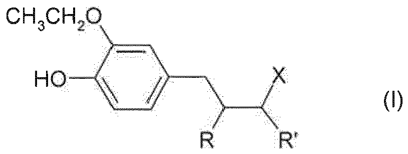 in which:
- R represents a hydrogen atom or a saturated or unsaturated and linear or branched C1-C6 hydrocarbon radical;
- R' represents a saturated or unsaturated and linear or branched C1-C18 hydrocarbon radical, optionally substituted by a hydroxyl group;
- C-X represents C=O or CH-OH.

2. Method according to Claim 1, in which R represents H, methyl or ethyl.

3. Method according to either of the preceding claims, in which R' represents a saturated linear C1-C6 or unsaturated linear C2-C6 hydrocarbon radical, optionally substituted by a hydroxyl group.

4. Method according to one of the preceding claims, in which the compounds correspond to the formula (I), in which:
- C-X represents C=O, R = H and R' represents a linear C1-C6 alkyl radical, optionally substituted by an OH; preferably, R ' = methyl or ethyl; or else
- C-X represents CH-OH, R = H and R' represents a linear C1-C6 alkyl radical, optionally substituted by an OH; preferably, R' = methyl or ethyl.

5. Method according to one of the preceding claims, in which the compounds of formula (I) are chosen from the following compounds: 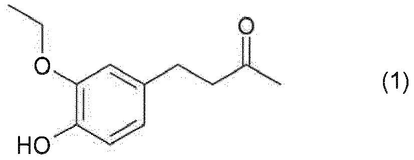 4-(3-ethoxy-4-hydroxyphenyl)butan-2-one 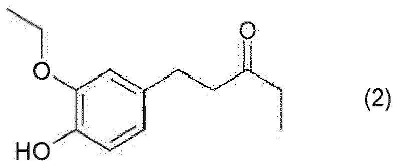 1-(3-ethoxy-4-hydroxyphenyl)pentan-3-one 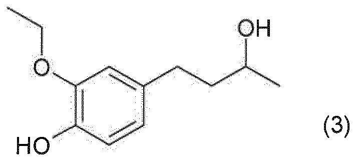 2-ethoxy-4-(3-hydroxybutyl)phenol.

6. Method according to Claim 5, in which the compound of formula (I) is 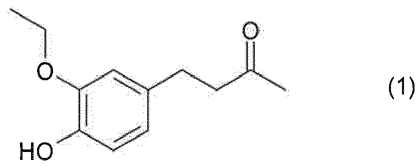 4-(3-ethoxy-4-hydroxyphenyl)butan-2-one.

7. Method according to one of the preceding claims, in which the compound of formula (I), alone or as a mixture, is present in a proportion of from 0.1% to 10% by weight, in particular from 0.5% to 5% by weight, with respect to the total weight of the composition.

8. Method according to any one of the preceding claims, in which the cutaneous aesthetic defects are chosen from skin imperfections due to hyperseborrhoea, and/or a scalp disorder.

9. Method according to any one of the preceding claims, in which the cutaneous aesthetic defects are chosen from a thick skin texture, glistening or shiny skin, skin exhibiting dilated follicular orifices or pores, skin exhibiting follicular orifices or pores filled with horny spicules or with comedones, rough skin or skin exhibiting an irregular relief, or skin exhibiting an impairment of the complexion.

10. Cosmetic use of at least one compound of formula (I) as defined in one of Claims 1 to 6, as agent for treating and/or preventing greasy skin or greasiness-prone skin and/or the associated cutaneous aesthetic defects.

11. Use according to the preceding claim, in which the cutaneous aesthetic defects are as defined in either of Claims 8 and 9.

12. Use according to either one of Claims 10 and 11, where the compound of formula (I) is present in a composition comprising a cosmetically acceptable medium.
